# EUROPEAN PATENT APPLICATION

(11) **EP 2 186 827 A1**
(43) Date of publication of application: **19.05.2010**
(21) Application number: 08019964.9
(22) Date of filing: 14.11.2008
(51) Int. Cl.: C07K 16/00, C12Q 1/68

(54) **Surrogate marker directed cDNA cloning of selectively induced mRNAs**

(71) Applicant: HS LifeSciences Ltd., 8008 Zürich (CH)
(72) Inventor: Stuart, Edward, 40489 Düsseldorf (DE); Esslinger, Christoph, 8032 Zürich (CH)
(74) Representative: Neuefeind, Regina

(57) **Abstract**

Provided is a method for cDNA cloning of specifically induced mRNAs from single cells using a novel type of genotype/phenotype coupling. In particular, the method enables the selective cloning of a specific mRNA encoding a novel phenotype from at least one single cell by determining a surrogate marker for the respective phenotype. A preferred embodiment is a method for surrogate marker directed cDNA cloning of an antigen induced mRNA sequence encoding the variable chain of an immunoglobulin, wherein the cDNA is cloned from a selected memory B cell by single cell RT-PCR.

## Description

### Field of the invention

The present invention relates to a method for the efficient enrichment of clones and the direct cloning of unknown gene products prior to their functional testing, in particular to the cloning of unknown gene products immediately after inducing their transcription. The present invention is particularly useful for preparing human monoclonal antibodies.

### Background of the invention

Human monoclonal antibodies are of pharmaceutical interest as candidates for immunotherapy in a variety of therapeutic indications. Current approaches comprise the immunization of transgenic mice that contain parts of the human immune system, or selection in vitro using human immune or non-human immune libraries (phage display). A direct approach to obtain a potentially valuable antibody would be by direct cloning of selected B cells from a human donor that expresses an antibody with a desired specificity. Antibodies from such a B cell clone would be most interesting if memory B cells would be used as the source because these cells bear the potential to produce affinity matured antibodies.

The typical methods established suffer from the drawback that they are not suitable to produce antibodies with the characteristics of those produced in the course of a physiological human immune response. Antibodies that occur naturally in humans either in response to an intrinsic pathogenic stimulus or exposure to an infectious agent may be directed against previously unidentified epitopes. Moreover, targets identified by such antibodies may be of higher therapeutic relevance than those antibodies that had been generated to targets that were selected based on scientifically biased assumptions about a potential therapeutic importance. In addition, the origin and maturation in a human subject of such human derived antibodies can be supposed to significantly decrease the probability of undesirable off-target reactivity and auto-toxicity in humans because the clinical history of the subject of origin can be selected to ensure the absence of undesirable side effects.

Techniques for the cloning of antibodies directly from a human subject have been described, these include the hybridoma technique or the EBV immortalization technique, or a combination of both (Steinitz et al., Nature 269 (1977), 420-422; Kozbor et al., J. Immunol. 127 (1981), 1275-1280 and Roder et al., Methods Enzymol. 121 (1986), 140-167). All these techniques suffer from the disadvantage of being of low efficiency. A more recent improvement of the EBV immortalization method has been described by Traggiai et al., Nature Medicine 10 (2004), 871-875. This method suffers from clonal instability most probably due to continued somatic hypermutation of antibody genes.

Therefore, an immunoglobulin providing system would be desirable that leads to the efficient cloning of somatically evolutionary optimized antibodies, in particular in case of polyclonal diseases, where various antibodies that are potentially acting in a synergistic manner are to be identified and the first cloning does not signify the end of the project. It is, for example, searched for evolutionary optimized antibodies from B memory cells from different patients whose condition, potentially immunologically induced, has made a positive progression. For this purpose, there is a need for entirely novel methods exhibiting a high efficiency while simultaneously being able to be miniaturized as well as automated.

The solution to this technical problem is provided by the embodiments characterized in the claims and described further below.

### Summary of the invention

The present invention generally relates to a method of selective cDNA cloning of induced mRNA, without the need to functionally measure the phenotype, or to directly measure the genotype. While the present invention is illustrated by embodiments where the cDNA of mRNAs encoding human monoclonal antibodies via the use of molecular beacons are cloned, the techniques described herein are not so limited.

The present invention is based on the observation that cloning of a specific mRNA of unknown sequence supposed to be present in a living cell can be achieved via beacon hybridization to (a) a known sequence segment which is coupled 1:1 to the unknown sequence, wherein the single cell-specific induction of the known sequence is directly functionally coupled with the phenotype of the unknown sequence, and/or (b) one or more surrogate markers reflecting an RNA expression pattern in the living cell as for the induction of the unknown mRNA sequence. The occurrence of both signals indicates the state of a B memory cell that expresses a corresponding immunoglobulin mRNA and that is activated by a specific antigen. In this context, induction of the expression of the unknown mRNA and concomitantly of the known sequence segment and surrogate marker(s), respectively, is performed by exposure of B lymphocytes, in particular memory B cells to an antigen of interest against which a specific antibody is desired to be cloned.

In principle, any desired antigen may be selected including but not limited to the group consisting of a human pathogen, toxin, chemical compound, allergen, tumor antigen, autoantigen, alloantigen or neoepitope of an otherwise physiological protein.

In a particular preferred embodiment of the method of the present invention the B lymphocyte is derived from a sample obtained from a subject who is symptom-free but affected with or at risk of developing a disorder, or a patient with an unusually stable disease course.

As described in the appended examples, the method of the present invention is preferably used for direct antigen selected cloning of a cDNA encoding the variable chain of an immunoglobulin specific for antigen of interest comprising the steps of:
(a) subjecting memory B cells to an antigen of interest;
(b) exposing said memory B cells to
   (i) a first molecular beacon which fluoresces upon hybridization with a first target RNA sequence encoding the constant region of an immunoglobulin; and/or
   (ii) at least one second molecular beacon which fluoresces upon hybridization with at least one second target RNA sequence encoding a marker for an antigen activated memory B cell;
(c) quantifying the level of fluorescence in said memory B cell, wherein the presence or increased or decreased level of fluorescence is indicative for the status of an antigen activated memory B cell;
(d) selecting an antigen activated memory B cell determined in step (c); and
(e) cloning the cDNA from the selected memory B cell by single cell polymerase chain reaction (scPCR).

In this embodiment as illustrated below the method of the present invention preferably does not involve transformation of cells in order to prolong their life span and/or providing clonal derivatives but is preferentially employed on a single cell level.

Subsequently, the cDNA may be cloned into an expression host cell in order to permit expression of the antibody of interest or immunoglobulin chain thereof in that host cell. Naturally, the cDNA can be manipulated to introduce restriction sites, to change codon usage, alter the amino acid sequence of the immunoglobulin chain while keeping antigen specific in kind and/or to add or optimize transcription and/or translation regulatory sequences.

In a further embodiment the method of the present invention comprises culturing the host cell under conditions where the antibody of interest is expressed; and optionally purifying the antibody of interest or immunoglobulin chain thereof.

Naturally, the present invention also extends to the antibody or equivalent antigen-binding molecule obtainable by the method of the present invention, which antibody is preferably a human antibody.

Further embodiments of the present invention will be apparent from the description and Examples that follow.

### Brief description of the drawings

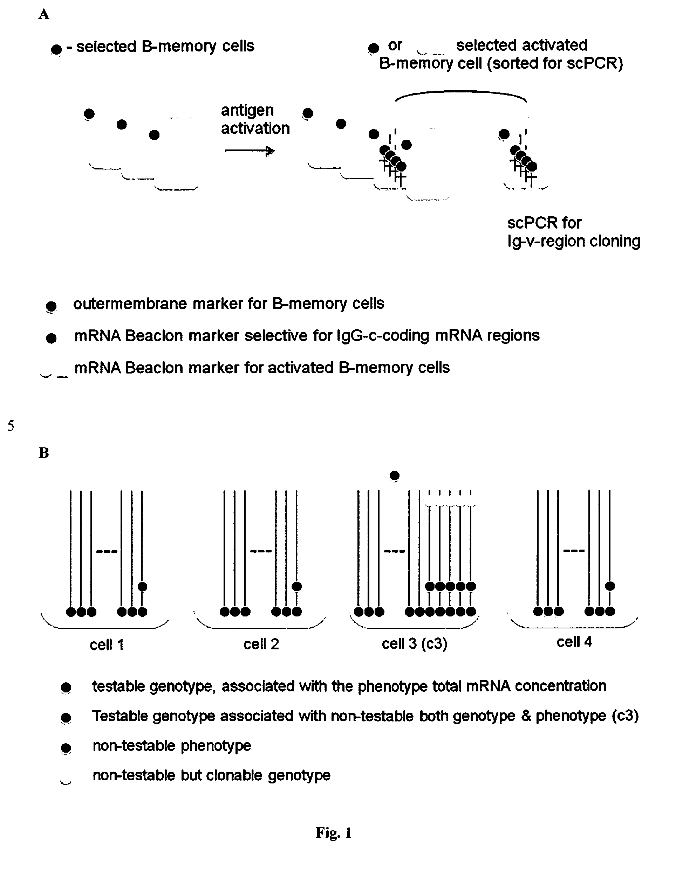

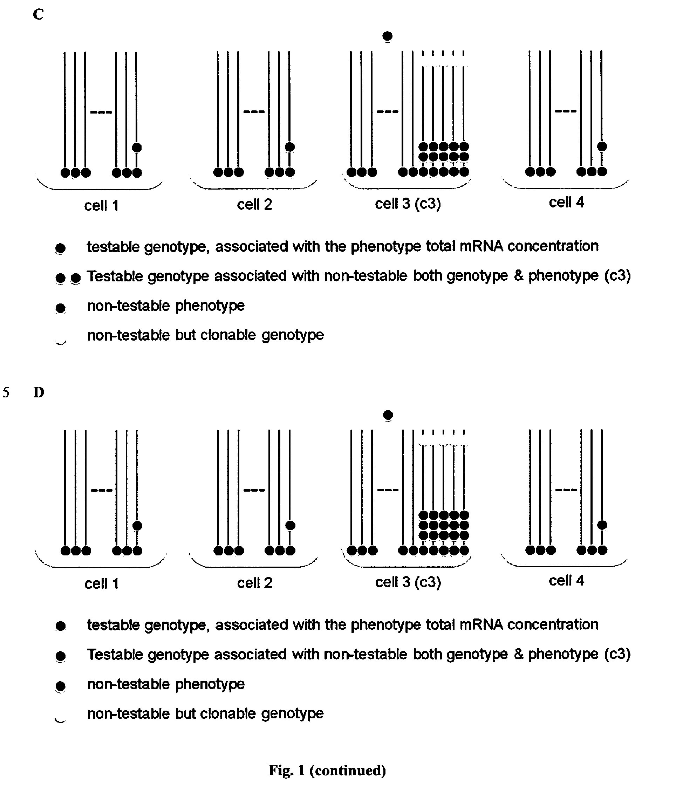

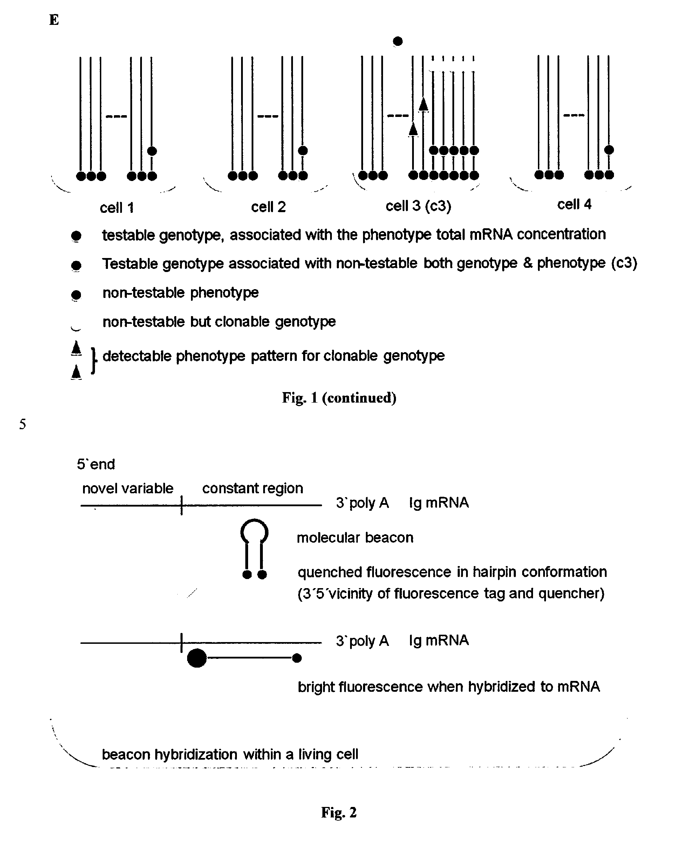

- **Fig. 1:**: (A) The method of the present invention is illustrated by direct cDNA cloning of an induced immunoglobulin variable chain encoding mRNA from selected antigen activated B-memory cells using single cell reverse transcriptase-polymerase chain reaction (scRT-PCR).
(B) Successful induction of a cell is understood to denote (i) conversion to the activated cell state and (ii) (clonal) induction of the transcription of the somatically varied gene locus from the corresponding combination of heavy and light chain. The activated state of the cell is determined by a surrogate marker, see cell 3, such as the constant region of an IgG with an appropriate probe, e.g., a molecular beacon; see also Figure 2. (At this point in time, the functionality of the Ig protein cannot yet be tested, at a later point in time the mRNA will not easily be clonable anymore due to apoptosis).
(C) and (D) A further embodiment of (B), wherein two or more probes are used to determine the surrogate marker, for example via fluorescence correlation spectroscopy. In this respect, the probes may be labeled with the same or different fluorescent molecules.
(E) In an associated manner, further known mRNAs are also induced and can be identified via a pattern analysis conducted according to the present invention.
- **Fig. 2:**: illustrates the use of a "molecular beacon", also designated by the abbreviation "MB" in accordance with the particularly preferred embodiment of the present invention. MBs are single-stranded oligonucleotide hybridization probes that under physiological conditions form a stem-and-loop structure (called hairpin) as the respective termini are complementary to each other. The total MB sequence is selected for maximum complementarity to a target RNA sequence selected as surrogate marker allowing for hybridization of such MBs to such RNA sequence in order to form a duplex structure such as with part of the mRNA sequence encoding the constant region of an immunoglobulin (Ig) associated with a novel yet unknown variable region. An MB is designed by a fluorophore covalently linked to one end of the MB and a quencher is covalently linked to the other end of the MB oligonucleotide sequence. Molecular beacons do not fluoresce when they are free in solution under physiological conditions. However, when they hybridize to e.g. the Ig mRNA they undergo a conformational change upon forming a duplex with the target sequence that enables them to fluoresce brightly as fluorescent dye and quenching moiety get separated compared to their close vicinity in the hairpin structure: In the absence of Ig mRNA, the probe is dark, because the stem places the fluorophore so close to the non-fluorescent quencher that they transiently share electrons, eliminating the ability of the fluorophore to fluoresce. When the probe encounters an Ig mRNA of the constant region, it forms a probe-Ig mRNA hybrid that is longer and more stable than the stem hybrid. The rigidity and length of the probe-target hybrid precludes the simultaneous existence of the stem hybrid. Consequently, the molecular beacon undergoes a spontaneous conformational reorganization that forces the stem hybrid to dissociate and the fluorophore and the quencher to move away from each other, restoring fluorescence. The signal intensity of the beacon hybridization, which is a measure for the Ig mRNA concentration, can be calibrated and utilized for optimal cloning efficiency. Subsequently to the detection of fluorescence, said individual cell is deposited for single cell-based PCR of the variable chains. The combinations of thus cloned light and heavy chains from different single cell clones can also be measured in corresponding expression cloning systems.

### Detailed description of the invention

The present invention generally relates to a selective cloning technology using a novel type of genotype/phenotype coupling. Generally, the method of the present invention enables the selective cloning of specific novel mRNA, i.e. cDNA encoding a novel phenotype from at least one single cell by determining a surrogate marker for the respective phenotype. Preferably, said phenotype is a qualitative or quantitative RNA pattern of known target RNA sequences of at least one such cell, wherein such qualitative or quantitative RNA pattern is characteristic for such novel phenotype. Typically, said phenotype is associated with the expression of such novel mRNA into a novel protein or peptide which may be determined by immunological or functional assays.

In one embodiment of the method of the present invention the desired mRNA is selectively associated with or contains at least one known sequence motif. This embodiment takes advantage of mRNAs which are generated by, for example differential splicing or exon shuffling of the underlying genes they are transcribed from. Thus, a given mRNA may contain yet unknown sequences such as variable sequences of an immunoglobulin or immunoglobulin-like molecule and known sequences such as the constant region of the mentioned immunoglobulin or immunoglobulin-like molecule.

Hence, the novel and inventive approach of the present invention is particular suited for the cloning of antibodies that are specific for an antigen of interest, e.g. an antigen of pathological implication from human memory B cells. More specifically, antigen-reactive memory B cells are identified by mimicking in vitro the activation of memory B cells as it occurs in the body during a recall response by re-exposure to the cognate antigen. Upon exposure to antigen the signaling cascade induced in memory B cells will be detected by determining corresponding surrogate markers. In principle, detection of surrogate markers can be achieved via various ways, for example visualization by histological assays such as for transcription factor activation, e.g. the translocation of NFkB into the nucleus; staining of up-regulated cell surface receptors such as CD38 and CD138; monitoring of phenotypic changes upon differentiation into plasma cells, e.g. size, proliferation or markers reflecting the same, and preferably by detecting a representative target RNA sequence, RNA pattern or profile. In this respect, the detection of up-regulation of mRNA transcripts induced by B cell receptor (BCR)/surface immunoglobulin (sIg) cross-linking in a time and quantity resolved fashion is preferred, for example of RNA transcripts encoding TNF-α, LT, CD38, CD138, IkB or the constant region of IgG. Typically, labeled oligonucleotide probes complementary to at least part of the surrogate target RNA transcript(s) are used. Most preferably, molecular beacons are used as labeled oligonucleotide probes.

Molecular beacons (MBs) are well known to the person skilled in the art and illustrated in the appended Example 3 and Figure 2 as well as in the cited literature. Thus, the molecular beacon technology enables the sensitive quantification of mRNAs in living cells either being fixed to a solid support or living cells being in suspension. In case of living cells such cells can subsequently be cultivated. The present invention makes use of this technology in order to analyze cells for the appearance of a specific somatically altered and optimized gene coupled to direct selection and cloning of such gene or corresponding mRNA via single cell (RT-PCR). The technology in accordance with the present invention pertains to the cloning of a specific novel phenotypic and detectable property encoded by an otherwise unknown mRNA which mere presence is identified by means of such molecular beacon selective to detect at least one Ig heavy or light chain coding sequence.

Cells of the optimum status for RT-PCR based cloning from single cells can be selected. Later on in expression cloning, the same molecular beacon alone or in combination with other such molecular beacons can be used as tools for identifying a cell type of stable and high yield expression of a gene functionally coupled to such desired phenotype, e.g. positive immunoreactivity in an ELISA assay or functional assay and the like. In case of Abeta specific binding molecules the TAPIR assay may be used as described in international application WO2004/095031, the disclosure content of which is incorporated herein by reference.

Hence, the present invention generally relates to a method for cloning a cDNA of an mRNA sequence encoding a polypeptide of interest (POI) comprising the steps of:
(a) providing cells potentially capable of expressing said mRNA;
(b) exposing said cells to an agent capable of inducing the expression of said mRNA in said cell;
(c) determining a surrogate marker for cells expressing said mRNA;
(d) isolating at least one cell which displays said surrogate maker; and
(e) cloning the cDNA of said mRNA sequence from the cell.

Put in other words, the present invention pertains to a method of selective cDNA cloning of specifically induced mRNAs encoding a novel functional phenotype from at least one single cell without the detection of the functional phenotype of such specific mRNA or the respective genotype, but by measuring a surrogate marker for the respective phenotype.

In accordance with the present invention said surrogate marker is other than the immunological or biological activity of said POI. Nevertheless, the presence, absence or altered level of the predetermined surrogate marker is indicative for a cell expressing the desired mRNA. Accordingly, the surrogate marker may be qualitatively and/or quantitatively determined.

The surrogate marker(s) to be used in accordance with the present invention may be of any kind, for example a protein selected from the group consisting of a cell surface-localized protein, e.g., a cell surface receptor, or an intracellular protein, e.g., a transcription factor. In context with cloning of antibodies that are specific for an antigen of interest, e.g. an antigen of pathological implication from human memory B cells said cell surface receptor is preferably CD138, and said transcription factor induced protein is IkB. Accordingly, in this embodiment the surrogate marker is preferably indicative for the status of an antigen activated lymphocyte. However, other surrogate markers for different embodiments are known to the person skilled in the art as well. For example, in close analogy to CD138, CD38, an additional plasma cell marker, has been shown to be upregulated during the transition of memory B cells to plasma cells in vitro as described by Arpin et al., Journal of Experimental Medicine 186 (1997), 931-940. Likewise, crosslinking of the B cell antigen receptor (BCR) in vitro resulted in transcription factor induced secretion of the cytokines TNF-alpha and LT-alpha as described by Duddy et al., Journal of Immunology 178 (2007), 6092-6099.

One particular advantage of the method of the present invention is that the desired mRNA and POI may hitherto be unknown. This is for example almost always the case for antibodies intended to be cloned from a memory B cell but may also apply to other classes of proteins, for example tumor suppressors, antiviral proteins such as interferons, or tissue and development specific expressed proteins which are supposed to be expressed and present in a cell after induction because of the phenotype of the cell but hitherto have not determined and cDNA could not be cloned. On the other hand, the POI may be known per se but not in context with the cell under investigation and being exposed to the agent in accordance with the method of the present invention.

Hence, in principle any cell may be used in the method of the present invention which is supposed to express an mRNA of interest for the supposed presence of which a surrogate marker is known. Preferably, said cells are lymphocytes selected from the group consisting of memory B cells, B cells or T cells, most preferably the lymphocytes are human memory B cells.

As a further advantage, the method of the present invention is applicable and preferably designed to be performed on a single cell level. Hence, via the use of appropriate surrogate markers and detection means, for example molecular beacons as described in detail below, it is possible to analyze cells prior to their becoming instable, for example due to the initiation of apoptosis, and clone the cDNA via single cell RT-PCR. Accordingly, the method of the present invention does not require proliferation of the cells and establishing a cell line. This aspect is particular relevant in the preparation of human antibodies from B cells, which hitherto requires immortalization and clonal expansion of the cell.

Thus, in view of the above, the present invention is preferably applied to the cloning of cDNAs encoding a POI selected from the group consisting of antibodies and antibody-like molecules, in particular cDNAs encoding the variable domain of an antigen binding molecule belonging to the immunoglobulin supergene family. The group of molecules known as the immunoglobulin supergene family include immunoglobulins, T-cell receptors, CD4, CD8, Class I and Class II Major Histocompatibility Complex (MHC) MHC molecules and others. Preferably, POI essentially consists of or contains a fragment of the variable light or variable heavy chain of an antibody.

For the sake of illustration, the method of the present invention is further described for the cloning of cDNAs encoding the variable domain of human monoclonal antibodies from memory B cells, the techniques described herein however are not so limited.

In particular, the method of the present invention greatly facilitates the cloning of human, in particular human subject-derived antibodies. Applied to selected clinical responders it is expected that the method of the present invention will lead to the identification and isolation of novel candidate antibodies for the immunotherapy of various diseases as well as a means for the isolation of novel disease associated antigens which because of the selectivity and specificity of the method of the present invention may be more reliable for use as clinical markers and targets for therapeutic intervention.

The cells, preferably B lymphocytes to be used in accordance with the present invention can be obtained from various sources (e. g. from whole blood, from peripheral blood mononuclear cells (PBMCs), from blood culture, from bone marrow, from organs, etc.), and suitable methods for obtaining human memory B cells are well known in the art. Samples may include cells that are not memory B cells e.g. other blood cells. A specific human memory B lymphocyte subpopulation exhibiting the desired antigen specificity may be selected before subjected to the agent in step (b) of the method of the present invention by using methods known in the art.

In a preferred embodiment of the method of the present invention said agent is or comprises an antigen. In principle, any desired antigen may be selected including but not limited to the group consisting of a human pathogen, toxin, chemical compound, allergen, tumor antigen, autoantigen, alloantigen or neoepitope of an otherwise physiological protein. Antigens of interest are disclosed for example in international application WO2004/076677. In one embodiment the agent is of cellular origin and, for example, may comprise cells or a subfraction of certain cells, e.g. cell membranes displaying an epitope of interest on their cell surface.

Rescreening of the resultant cDNA and its encoded POI, respectively, may be performed according to methods well known in the art, for example immunological assays such as ELISA using the same antigen the cell has been exposed to or an epitope thereof and the like or functional assays depending on the biological activity suspected for the for the POI. Regarding the characterization, identification and isolation of neo-epitope and tumor antigen specific human antibodies the present specification is specifically supplemented with the teaching provided by international application WO2008/081008 and W02008/110373. In particular, the method for validating and producing neo-epitope specific diagnostically and therapeutically useful binding molecules essentially as disclosed in international application WO2008/081008 may be employed but altered on the level of B cell selection and treatment as disclosed in the present application.

Furthermore, regarding the isolation, molecular cloning and recombinant production of patient-derived human antibodies the present specification is supplemented with the molecular cloning step using single cell RT-PCR and the re-screening of recombinant antibody clones with tissue microsections as disclosed in WO2008/110373. Likewise, the method for validating and producing tumor-specific diagnostically and therapeutically useful binding molecules, in particular human antibodies that are directed against antigens associated with tumor cells and tissue essentially as disclosed in international application WO2008/110373 may be employed but again altered on the level of B cell selection and treatment as disclosed in the present application.

Hence, in a particular preferred embodiment of the method of the present invention the cells, preferably B lymphocytes and most preferably memory B cells are derived from a sample obtained from a subject who is symptom-free but affected with or at risk of developing a disorder, or a patient with an unusually stable disease course or effectively suppressing the manifestation or outbreak of a disorder. This embodiment is a further development of the corresponding methods of obtaining patient and disease specific human antibodies disclosed in international applications WO2008/081008 and W02008/110373.

As already mentioned above, molecular beacons are preferably used for the determination of the surrogate marker(s). Accordingly, in this embodiment said surrogate marker is the presence or altered level of at least one target RNA sequence encoding the above described surrogate marker(s). In context with the cloning of immunoglobulins said target RNA sequence preferably encodes I Kappa B (IkB), CD38, or CD138, TNF-alpha or LT-alpha; see also the examples. Alternatively, or in addition a target RNA sequence may be selected encoding the constant region of an immunoglobulin or immunoglobulin-like molecule, preferably wherein said immunoglobulin is the light or heavy chain of an antibody; see also Figure 1 and 2.

In this embodiment of the present invention, as illustrated in the examples and figures, step (c) comprises in situ hybridization of said target RNA sequence in the cell with at least one labeled sequence, typically an oligonucleotide sequence, which is complementary to at least part of said target RNA sequence. In a particular preferred embodiment, the method of the present invention comprises in situ hybridization of a first target RNA sequence encoding the constant region of an immunoglobulin or immunoglobulin-like molecule and at least one second RNA target sequence which is indicative for the status of an antigen activated lymphocyte. Most preferably, said labeled sequence is a molecular beacon which fluoresces upon hybridization to said target RNA sequence.

A molecular beacon is a nucleic acid probe that recognizes and reports the presence of a specific nucleic acid sequence. The probes are nucleotide acid sequences or derivatives thereof with a central stretch of nucleotides complementary to the target sequence, and termini comprising short mutually complementary sequences able to form hairpin stem structures. One terminus is covalently bound to a fluorophore and the other to a quenching moiety. When in their native state with hybridized termini, the proximity of the fluorophore and the quencher is such that no fluorescence is produced. The beacon undergoes a spontaneous fluorogenic conformational change when hybridized to its RNA target nucleic acid; see, for example, US patent no 5,925,517. This property has enabled researchers to detect specific nucleic acids primarily in in vitro reactions and in some cases even in living cells. In-site visualization of messenger RNA has been achieved using molecular beacons delivered to living cells in liposomes (Matsuo, Biochim. Biophys. Acta 1379 (1998), 178-184). Dual nucleic acid probes with resonance energy transfer moieties, in particular, fluorescent or luminescent resonance energy transfer moieties on hairpin stem-loop molecular beacon probes that hybridize sufficiently near each other on a subject target RNA sequence to generate an observable interaction are described international application WO03/000933, the disclosure content of which is incorporated herein by reference in its entirety).

One major advantage of those stem-loop probes is that they can recognize their targets with a higher specificity and sensitivity than the linear oligonucleotide probes. Accordingly, molecular beacons are particularly suitable for real-time monitoring of PCR and mRNA detection in living cells and thus are preferred probes in accordance with the present invention for qualitatively and/or quantitatively determining the surrogate marker according to step (c) of the method of the present invention

Fluorescence may be quantified, for example by fluorescence microscopy or fluorescence activated cell sorter technology. Thus, the cell in step (d) of the method of the present invention may be isolated by flow cytometry technology, e.g., comprising fluorescence activated cell sorting. In vivo detection of nucleic acids via molecular beacons, in connection with the subsequent selection of cells is also described in international application WO2004/020625, the disclosure content of which is incorporated herein by reference.

In one embodiment the fluorescence is determined by fluorescence correlation spectroscopy (FCS). Intranuclear diffusion and hybridization state of oligonucleotides measured by fluorescence correlation spectroscopy in living cells is described in the art; see, e.g., Politz et al., Proc. Natl. Acad. Sci. USA 95 (1998), 6043-6048, describing experiments wherein fluorescein-labeled oligodeoxynucleotides were introduced into cells, and their molecular movements inside the nucleus were studied by FCS and fluorescence recovery after photobleaching (FRAP).

FCS analyzes minute fluorescence-intensity fluctuations about the equilibrium of a small ensemble (<10³) of molecules and provides precise information about a multitude of measurement parameters, including diffusion coefficients, local concentration, states of aggregation and molecular interactions. A review and step-by-step guide to the application of FCS to cellular systems, including methods for minimizing artifacts, optimizing measurement conditions and obtaining parameter values in the face of diverse and complex conditions of the living cell is described Kim et al., in Nat. Methods 4 (2007), 963-973 with a discussion of advantages and disadvantages of one-photon versus two-photon excitation for FCS available in Supplementary Methods online.

Accordingly, FCS is particularly suited for the detection of different probes for more than one surrogate marker and/or of multiple probes for the same surrogate marker, preferably wherein said probes are labeled with different fluorescent molecules.

Thus, in one preferred embodiment of the present invention at least two oligonucleotide sequences are used for detecting the target RNA sequence(s), which are labeled with different or identical types of fluorescent markers. In this context, said at least two oligonucleotide sequences may be complementary to at least part of different target RNA sequences or to different parts of the same target RNA sequence. Accordingly, each such fluorescently labeled type of oligonucleotide may be attached to a fluorescent marker which can be differentiated from another fluorescent marker, for example by different color of emission or excitation, different life time or the status of excitation or brightness.

Corresponding means and methods for FCS are well known to the person skilled in the art. For example, a method for the identification of one or a small number of molecules by using laser-stimulated FCS is disclosed in international application WO94/16313. Furthermore, international application WO96/013744 describes a method of determining substance-specific parameters of one or a plurality of molecules by correlation spectroscopy. A method for characterizing samples having fluorescent particles comprising inter alia the steps of exciting particles in a measurement volume to emit fluorescence by a series of excitation pulses and monitoring the emitted fluorescence by detecting sequences of photon counts is described in international application WO01/059436. A similar method which is well suited for single molecule observations is performed by the detection of fluorescence or Raman signal from single molecules by photon counting as described in international application WO02/050516. Another method for analyzing properties of a sample by measuring fluorescence parameters is described in international application WO02/050516 WO2004/036195, using multi-parameter fluorimetric analysis in a massive parallel multi-focal arrangement.

In this context, it is understood that while the detection of the surrogate marker(s) in accordance with the method of the present invention is illustrated and preferably performed on the RNA level detection on the protein level is applicable as well. For example, a method for testing a substance interacting with a target molecule in a cell by FCS by determining a distribution of diffusion coefficients for the fluorescently labeled target molecule such as a nuclear hormone receptor is described in international application WO2006/013109. Furthermore, international application WO2006/037622 describes the detection of two individual autofluorescent proteins, which differ from each another in at least one detectable photophysical parameter such as different emission wavelengths, different fluorescence life times or different polarizations, for detecting protein-protein interactions by fluorescence cross correlation spectroscopy (FCCS) in vivo.

All the above described methods and the corresponding disclosure content of the cited documents are incorporated herewith by reference.

As illustrated in Examples 3 and 4 steps (d) and (e) of the method of the present invention are advantageously performed at a point of time wherein the total mRNA in the cell remains substantially intact and able to be cloned. Typically, the cloning of the cDNA in step (e) is performed by single cell polymerase chain reaction (PCR) which is preceded by reverse transcription (RT) of the respective mRNA; see also the Examples. Thus, single cell Ig PCR is conducted at a point in time when the antigen induction of preselected native B memory cells is triggered. In this context, according to the present invention, antigen-induced Ig mRNA induction is understood to denote (a) recognizing at least one co-expression of a beacon-specific mRNA 1:1 quantitatively or in form of a pattern together with at least one further RNA as surrogate marker coupled to the variable unknown but amplifiable sequence of the antigen-specific heavy or light chain, which are coupled in a coexistent clonable manner within the individual cell. Cell selection, e.g., sorting is preferably performed, when the ratio of the number of induced mRNAs to the intactness of the mRNAs is optimal. The optimal point in time for clonability may be determined in accordance with the present invention in advance, for example preparatory experiments prior to the actual cloning experiment; see also Example 3.
This experimental set up allows the entire method to be automated and parallelized, and immortalizing the induced cells can be omitted. The 1:1 causal chain is inventively utilized starting with B memory cells from stabilized patients:

To that end, the cell, e.g., activated memory B cell is selected and the cDNA being cloned with primers selected for IgG cloning as described in international application WO2008/081008, the disclosure content of which is incorporated herein by reference. Further methods of cloning the variable sequences of immunoglobulins without primer sequences are disclosed in US patent no. 7,135,310, the disclosure content of which is incorporated herein by reference. Respective antibodies or functionally active antibody fragments can be expressed and tested for selective binding properties by methods well known in the art.

As mentioned hereinbefore, the method of the present invention is applicable to other POIs as well, for example disease or pathogen induced POIs. In order to facilitate cloning of the desired cDNA of POIs among others which are induced or present in the cell subtractive suppression hybridization (SSH) may be employed, i.e. wherein the mRNA or cDNA is saturated with cDNA obtained from the same kind of cell before exposed to the inducing agent. SSH technique is well known to the person skilled in the art and described, e.g., in international applications WO03/093501 and WO2005/014853, the disclosure content of which is incorporated herein by reference.

In a further embodiment, the present invention relates to a method of producing a POI comprising steps of the method of the present invention of cloning the cDNA encoding the POI and further comprising inserting the cDNA in an expression vector. However, in vitro transcription and expression of the POI is envisaged as well, for example in wheat germ extract. Typically this embodiment further comprises inserting the vector into an expression host cell in order to permit expression of the POI in the host cell. As already mentioned, the cDNA may be manipulated in between to introduce restriction sites, to change codon usage, and/or to add or optimize transcription and/or translation regulatory sequences. All these techniques are state of the art and can be performed by the person skilled in the art without undue burden. The host cell will be cultured under conditions where the POI is expressed; and usually subsequently the POI will be purified from the host cell or the culture medium. A mentioned, the method of the present invention is advantageously applied to the preparation of an antibody or an equivalent antigen binding molecule.

Hence, the present invention also pertains to the POI encoded by the cDNA obtainable by the method of the present invention and/or obtainable by the method of producing sad POI. As mentioned, the POI is preferably an antibody, immunoglobulin chain thereof or an equivalent antigen-binding molecule. Thus, the present invention provides an antibody and equivalent antigen-binding molecule obtainable by the method of the present invention, which antibody is preferably a human antibody having two polypeptide chains, wherein one or both of polypeptide chains has/have a human VDJ sequence. Monoclonal antibodies produced by the methods of the present invention may be further purified, if desired, using filtration, centrifugation and various chromatographic methods such as HPLC or affinity chromatography. Techniques for purification of monoclonal antibodies, including techniques for producing pharmaceutical-grade antibodies, are well known in the art.

Fragments of the monoclonal antibodies of the present invention can be obtained from the monoclonal antibodies so produced by methods that include digestion with enzymes, such as pepsin or papain, and/or by cleavage of disulfide bonds by chemical reduction. Antibody "fragments" include Fab, F(ab')₂ and Fv fragments. The present invention also encompasses single-chain Fv fragments (scFv) derived from the heavy and light chains of a monoclonal antibody of the invention, e.g. the invention includes an scFv comprising the CDRs from an antibody of the invention.

For the sake of clarity only and without restricting the scope of the present invention most of the embodiments are discussed herein with respect to human antibodies and antibody-like molecules which represent the preferred binding molecules for the development of therapeutic and diagnostic agents in accordance with the present invention. However, it is to be understood that as used in context of the present invention the term "antibody", and fragment thereof, may also refer to other POIs, i.e. non-antibody binding molecules including but not limited to hormones, receptors, ligands, major histocompatibility complex (MHC) molecules, chaperones such as heat shock proteins (HSPs) as well as cell-cell adhesion molecules such as members of the cadherin, integrin, C-type lectin and immunoglobulin (Ig) superfamilies.

Monoclonal antibodies are particularly useful in identification and purification of the individual polypeptides or other antigens against which they are directed. The monoclonal antibodies of the invention have additional utility in that they may be employed as reagents in immunoassays, radioimmunoassays (RIA) or enzyme-linked immunosorbent assays (ELISA). In these applications, the antibodies can be labeled with an analytically-detectable reagent such as a radioisotope, a fluorescent molecule or an enzyme. The monoclonal antibodies produced by the above method may also be used for the molecular identification and characterization (epitope mapping) of antigens recognized by protected individuals in complex pathogens such as plasmodia, the isolation of cross-reactive protective antibodies in the case of highly variable pathogens such as those found in HIV and for detecting pathogens and determining their variability.

Antibodies of the present invention can be coupled to a drug for delivery to a treatment site or coupled to a detectable label to facilitate imaging of a site comprising cells of interest, such as cancer cells. Methods for coupling antibodies to drugs and detectable labels are well known in the art, as are methods for imaging using detectable labels. Antibodies of the invention may be attached to a solid support.

Antibodies of the invention are preferably provided in purified form. Typically, the antibody will be present in a composition that is substantially free of other polypeptides, e.g. where less than 90% (by weight), usually less than 60% and more usually less than 50% of the composition is made up of other polypeptides.

Antibodies of the invention may be immunogenic in non-human (or heterologous) hosts e.g. in mice. In particular, the antibodies may have an idiotope that is immunogenic in non-human hosts, but not in a human host. Antibodies of the invention for human use include those that cannot be obtained from hosts such as mice, goats, rabbits, rats, non-primate mammals, etc. and cannot be obtained by humanization or from xeno-mice.

Antibodies of the invention can be of any isotype (e.g. IgA, IgG, IgM, i.e. α, γ or µ heavy chain), but will generally be IgG. Within the IgG isotype, antibodies may be of the IgG1, IgG2, IgG3 or IgG4 subclass. Antibodies of the invention may have a κ or λ light chain.

The antibody of the present invention advantageously displays particularly high binding affinity with an equilibrium dissociation constant (KD) of the interaction with its cognate antigen in the lower nanomolar range. Preferably, the binding affinity of the binding molecule of the present invention with its cognate antigen is about at least 10⁻⁷M, more preferably at least 10⁻⁸M, particularly preferred 10⁻⁹M and still more preferred at least 10⁻¹⁰M.

The present invention also provides a pharmaceutical and diagnostic, respectively, pack or kit comprising one or more containers filled with one or more of the above described ingredients, i.e. POI, in particular antibody or equivalent binding molecule derived thereof, or corresponding means for their production and/or delivery, for example a polynucleotide, particularly vector encoding the antibody or a cell containing the same. Associated with such container(s) can be a notice in the form prescribed by a governmental agency regulating the manufacture, use or sale of pharmaceuticals or biological products, which notice reflects approval by the agency of manufacture, use or sale for human administration. In addition or alternatively the kit comprises reagents and/or instructions for use in appropriate diagnostic assays.

The pharmaceutical compositions of the present invention can be formulated according to methods well known in the art; see for example Remington: The Science and Practice of Pharmacy (2000) by the University of Sciences in Philadelphia, ISBN 0-683-306472. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Aerosol formulations such as nasal spray formulations include purified aqueous or other solutions of the active agent with preservative agents and isotonic agents. Such formulations are preferably adjusted to a pH and isotonic state compatible with the nasal mucous membranes. Formulations for rectal or vaginal administration may be presented as a suppository with a suitable carrier.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

The practice of the present invention will employ, unless otherwise indicated, conventional techniques of cell biology, cell culture, molecular biology, transgenic biology, microbiology, recombinant DNA, and immunology, which are within the skill of the art. For further elaboration of general techniques useful in the practice of this invention, the practitioner can refer to standard textbooks and reviews in cell biology and tissue culture; see also the references cited in the examples. General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Protein Methods (Bollag et al., John Wiley & Sons 1996); Non-viral Vectors for Gene Therapy (Wagner et al. eds., Academic Press 1999); Viral Vectors (Kaplitt & Loewy eds., Academic Press 1995); Immunology Methods Manual (Lefkovits ed., Academic Press 1997); and Cell and Tissue Culture: Laboratory Procedures in Biotechnology (Doyle & Griffiths, John Wiley & Sons 1998). Reagents, cloning vectors and kits for genetic manipulation referred to in this disclosure are available from commercial vendors such as BioRad, Stratagene, Invitrogen, Sigma-Aldrich, and ClonTech.General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11 (2001), 98-107.

### Example 1: Isolation of memory B cells from PBL

Memory B cells from preferably individual blood samples are enriched via cell sorting using cytometric sorting technology with the aid of the molecular markers CD19+, CD22+, CD27+, and CD80+. Herein, blood samples from preferably human individuals are employed, who are suspected to have already been in contact with the antigen or a neo-antigen of interest and have, via somatic variation, evolutionary optimized corresponding immunoglobulin with respect to their target-related efficiency and counter-selected for non-target-related or target-related toxicity; see also international applications WO2008/081008 and WO2008/110373.

For example, memory B cells are isolated with a two step selection protocol using the pan B cell marker CD22 and the memory B cell marker CD27 and the activation marker CD80 as a positive selection criteria, combined with negative selection of antigen-inexperienced B cells that expressed IgM, IgD. Selection of B cells from the bulk of PBL can be performed using the MACS technology and CD22 microbeads (Miltenyi, Bergisch Gladbach, Germany). PBL are labeled with MACS anti human CD22, FITC-labeled mAbs anti CD27, phycoerythrin-conjugated mAbs anti human IgD, CyChrome/PerCP-conjugated mAbs anti CD80 and APC-conjugated antibodies anti human IgM, CD3, CD8, CD56 (Becton Dickinson, Basel, Switzerland). CD22-positive cells can be isolated using LS columns and the Midi MACS device (Miltenyi) followed by selection of FITC- and CyChrome/PerCP-positive cells and phycoerythrin- and APC-negative cells using a MoFlo cell sorter (Dako, Fort Collins, USA). CD22-positive, CD27-positive, CD80-positive and IgM-, IgD-negative B cells can then be incubated. With this technique, approximately 10.000 to 100.000 memory B cells can be obtained from 30 ml of human blood.

Isolation of single B lymphocytes by cytometric cell sorting for amplification of human immunoglobulin heavy and light chains by semi-nested RT-PCR is described by Coronella et al., Nucleic Acids Res. 28 (2000), e85, published online; see section MATERIALS AND METHODS, which is incorporated herein by reference in its entirety, and can be adapted to the method of the present invention. Thus, prior to the amplification of the human immunoglobulin heavy and light chains from the single B lymphocytes or plasma cells the cells are exposed to the inducing agent with subsequent determining an appropriate surrogate marker; see Examples 2 and 3, infra.

### Example 2: Subjecting memory B cells to an antigen of interest

The memory B cells are stimulated by means of at least one specific antigen in culture medium with factors complementing for T cell help, for example CD40L, irradiated allogeneic T cells or irradiated mouse L cells stably transfected with CD40L.
For example, 100.000 memory B cells are incubated in 1ml culture medium with antigen or (suspensions of tumors that presumably contain the antigen of interest). Subsequently, the cells are incubated on CD40L transduced fibroblast cells or on CD40L coated cell culture dishes prior to transfection with molecular beacons.

In this state, activated memory B cells are viable for only a very short period of time. Besides CPG stimulation, typical methods simultaneously provide an EBV-mediated immortalization. The method of the present invention can dispense therewith because the state of the induction of the mRNA is immediately indicated, see Example 3, and the cell can be selected prior to undergoing apoptosis in order to subject the mRNA to amplification by means of single cell PCR (scPCR).

### Example 3: Surrogate marker mRNA detection in living memory B cells

Besides inducing the expression of the antigen specific immunoglobulin (heavy and light chain), the activated B cells are characterized by the induced expression of further proteins that are characteristic for the state of an antigen-activated B memory cell, e.g.CD38 and CD138 or TNF-alpha and LT-alpha; see Figure 1.

Said induction of the general activation markers can be followed either intracellularly on RNA transcript level, preferably via at least one beacon hybridization, or on the level of the translated protein with preferably extracellular detection. The transcription induction of the Ig-specific loci of the somatically optimized mRNA is followed by means of intracellular hybridization of at least one beacon within the living cell. The hybridization event is perceived in form of activating a fluorescence that can be recorded in a cytometer cell sorter.

For example, an antigen specific activation signature, i.e. a shift from memory B cell to plasma cell phenotype can be detected by a time resolved determination of the up-regulation of the mRNA of activation markers IkB, TNF-alpha, LT-alpha and the plasma cell markers CD38, CD138 and preferably as an additional marker the constant region of IgG (M,A,E), thereby identifying and selecting antigen-activated memory B cells.

Molecular signatures distinguishing human central memory from effector memory CD8 T Cell subsets are described in Willinger et al., J. Immunol. 175 (2005), 5895-5903. Systematic comparison of gene expression between memory and naive B cells demonstrating that memory B cells have unique signaling capabilities that can be followed is also described by Tomayko et al. in J. Immunol. 181 (2008), 27-38, the disclosure content of which is incorporated herein by reference. Transcriptional profiling of antigen-dependent B cell differentiation and memory formation that may be applied in accordance with the present invention in order to provide an RNA transcript profile for human antigen activated memory B cells is described by Bhattacharya et al. in J. Immunol. 179 (2007), 6808-6819, the disclosure content of which is incorporated herein by reference in its entirety.

Thus, cells are transfected with molecular beacons for IkB, CD38, CD138, TNF-a, and/or LT-a, and alternatively or preferably in addition for IgG. The nucleotide sequences of the mentioned surrogate markers are well known in the art and can be easily retrieved from EMBL-BANK with the tools available at the web site of The European Bioinformatics Institute (EBI) which forms part of the European Molecular Biology Laboratory (EMBL) or from GenBank hosted by the National Center for Biotechnology Information (NCBI). Accession numbers of reference nucleotides sequences are as follows: IkB: AK313421; TNF-a: A21522; LT-a: X01393;CD38: NM_001775;CD138: NM_002997; and IgG-constant region: J00228; see also Takahashi et al., Cell 29 (1982), 671-679.

Molecular beacons (MBs) for detecting IkB, TNF-a, LT-a, CD38, CD138, and IgG-constant region can be designed based on the specific nucleotide sequences identified by bioinformatics. MBs have typically 23 nucleotides with a hairpin loop usually made up to have 5 or 6 (most of time 5) base pairs and loop sequences complementary to a unique nucleotide sequence of the target RNA. A general method for making a MB is disclosed by Peng et. al., Cancer Res. 65 (2005), 1909-1917. The MBs can then synthesized by a contractor, for example MWG Biotech, Inc. located in North Carolina.

For example, one MB specific for the constant region of an IgG may have a stem of 5 nucleotides and the 5 '-end labeled with FITC and the 3 '-end labeled with a quencher, Dabcyl. A second MB specific for another part of the constant region of the IgG or for another surrogate marker, e.g., CD 138 may have a stem containing 6 nucleotides with the 5 '-end labeled with Texas-red and the 3'-end with Dabcyl. Both MBs only generate fluorescent signals when hybridized to their specific target RNA. Of course, three and more MBs which are specific for the same or different surrogate markers may be used as well.

The specificity of the MBs may be tested in vitro, for example within a mixture of various synthesized DNA targets including samples containing the desired target RNA and DNA, respectively, as well as negative controls. The fluorescence units can be measured using a fluorescence microplate reader. MBs for subsequent use will only bind and generate strong fluorescent signal when mixed with samples containing their specific DNA target.

In order to identify the most suitable time window for selection of the cell expressing the POI and in which the cloning efficiency is optimal the corresponding mRNA is quantified by fluorescence analysis (cytometer sorter) at time points 0 min, 30 min, 1h, 2h, 5h, and 12h after exposure to the agent. In addition, mRNA of house-keeping genes may be analyzed in order to assess whether the total RNA of the cell remains substantially intact or degradation due to, e.g., apoptosis already started. The principle use of molecular beacons for detecting the level of gene expression in a sample of cells is known to the person skilled in the art; see, e.g., international application WO2006/060561, in particular at paragraphs [0012] and [0045] to [0048] as well as the example and references cited therein, the disclosure content of which is incorporated herein by reference in its entirety. Furthermore, see US patent application US 2008/032282, in particular the examples concerning the use of molecular beacons in expression profiling and the means and methods therefore, the disclosure content of which is incorporated herein by reference in its entirety.

In addition, efficient, rapid and sensitive detection of RNA in living cells using peptide, i.e. TAT peptide-linked molecular beacons that possess self-delivery, targeting and reporting functions is described in Nitin et al., Nucleic Acids Research 32 (2004), e58, published online. Using molecular beacon (MB)-fluorescent probes to image the viral genomic RNA (vRNA) of human RSV (hRSV) in live Vero cells is described in Santangelo and Bao, Nucleic Acids Research. 35 (2007), 3602-3611. The means and methods for delivering molecular beacons into a cell described in the cited literature may be applied to the method of the present invention as well.

Target cells are characterized by early presence of mRNA of IkB and TNF-alpha and LT-alpha and subsequent expression of CD138, CD38 and IgG; see Figure 1. The presence of the markers can be assayed simultaneously, quantitatively and in a time resolved fashion using molecular beacons with different colors. However, as only the cell recognizing the antigen is addressed, the group-specific identification of different classes of Ig is sufficient for identifying the cell bearing the non-testable somatically varied light and heavy chains via a corresponding beacon hybridization to constant sequence segments; see also Figure 1A and 2. Cells fulfilling the criteria of an early activation and subsequent initiation of a plasma cell phenotype are sorted or selected as single cells using a cytometer cell sorter. For example, if the cells have not already been provided as single cells during their initial isolation, see reference to Coronella (2000), supra, the cells are singled out by single cell deposition into 96 well plates using a cytometer cell sorter (MoFlo, Dako, Fort Collins, USA), the device being set to deposit one single cell per well directly in 96 well plates. The cells can either be directly subjected to single cell RT-PCR and the specificity of the antibody be tested upon recombinant expression or, alternatively the cells may be cultured under appropriate supportive culture conditions to allow for the secretion of antibody the specificity of which can then be tested prior to its molecular cloning by RT-PCR.

### Example 4: cDNA cloning from the selected memory B cell by single cell polymerase chain reaction (scPCR)

The individual cells (or the clonally expanded cells) are then subjected to RT-PCR of the Ig-variable regions of the antibody heavy light chains; see also Coronella (2000), supra, and international applications WO2008/081008 and WO2008/110373. To that end mRNA obtained from the cell is reverse transcribed and the Ig-heavy variable regions are amplified by PCR using frame work 1 and J-H-region specific primer mixes specific for all families of the human Ig-variable heavy chain. The sequence is then determined by cycle sequencing. After cloning into appropriate plasmid expression vectors and transformation into E.coli the sequence is re-analyzed prior to recombinant expression..

The specificity of the cloned variable region of an antibody is verified upon recombinant expression using vectors that provide the constant regions of immunoglobulin heavy and light chains. This directly provides the antibody of interest, which can be tested in immunological assays such as ELISA and the like or functional assays depending on the biological activity suspected for the antibody or generally protein of interest.

## Claims

1. A method for cloning a cDNA of an mRNA sequence encoding a polypeptide of interest (POI) comprising the steps of:
(a) providing cells potentially capable of expressing said mRNA;
(b) exposing said cells to an agent capable of inducing the expression of said mRNA in said cell;
(c) determining a surrogate marker for cells expressing said mRNA;
(d) isolating at least one cell which displays said surrogate maker; and
(e) cloning the cDNA of said mRNA sequence from the cell.

2. The method of claim 1, wherein
(i) said POI essentially consists of the variable domain of an antigen binding molecule belonging to the immunoglobulin supergene family, preferably wherein said POI comprises a fragment of the variable light or variable heavy chain of an antibody;
(ii) said cells are lymphocytes selected from the group consisting of memory B cells, B cells or T cells; preferably human memory B cells;
(iii) said agent contains at least one antigen, preferably selected from the group consisting of a human pathogen, toxin, chemical compound, allergen, tumor antigen, autoantigen, alloantigen or neoepitope of an otherwise physiological protein; and/or
(iv) said surrogate marker is indicative for the status of a cell induced to express said mRNA, preferably an antigen activated lymphocyte.

3. The method of claim 1 or 2, wherein said surrogate marker is the presence or altered level of at least one target RNA sequence.

4. The method of claim 3, wherein at least one target RNA sequence encodes Tumor nekrosis factor-alpha (TNF-alpha), Lymphotoxin-alpha (LT-alpha), I Kappa B (IkB), CD38, or CD138.

5. The method of claim 3 or 4, wherein at least one target RNA sequence encodes the constant region of an immunoglobulin or immunoglobulin-like molecule.

6. The method of any one of claims 3 to 5, wherein step (c) comprises in situ hybridization of at least one target RNA sequence in the cell with at least one labeled oligonucleotide sequence which is complementary to at least part of said target RNA sequence; preferably comprising in situ hybridization of a first target RNA sequence encoding the constant region of an immunoglobulin such as the light or heavy chain of an antibody or of an immunoglobulin-like molecule and at least one second RNA target sequence which is indicative for the status of an antigen activated lymphocyte.

7. The method of claim 6, wherein said at least one oligonucleotide sequence is a molecular beacon which fluoresces upon hybridization to said target RNA sequence.

8. The method of claim 6 or 7, wherein at least two oligonucleotide sequences are used which are labeled with different or identical types of fluorescent markers, wherein said at least two oligonucleotide sequences are complementary to at least part of different target RNA sequences or to different parts of the same target RNA sequence.

9. The method of claim 7 or 8, wherein fluorescence is quantified by fluorescence microscopy or fluorescence activated cell sorter technology.

10. The method any one of claims 1 to 9, wherein the cell in step (d) is isolated by flow cytometry technology.

11. The method of any one of claims 1 to 10, wherein the cloning of the cDNA in step (e) is performed by single cell reverse transcription polymerase chain reaction (scRT-PCR).

12. A method of producing a POI comprising steps of the method of any one of claims 1 to 11 and further comprising inserting the cDNA in an expression vector and preferably further comprising inserting the vector into an expression host cell in order to permit expression of the POI in a host cell and culturing the host cell under conditions where the POI is expressed; and optionally purifying the POI.

13. A POI encoded by the cDNA obtainable by the method of any one of claims 1 to 11, or obtainable by the method of claim 12.

14. The POI of claim 13, which is an antibody, immunoglobulin chain thereof or an equivalent antigen-binding molecule.

15. A method for surrogate marker directed cDNA cloning of an antigen induced mRNA sequence encoding the variable chain of an immunoglobulin comprising the steps of:
(a) subjecting memory B cells to an antigen of interest;
(b) exposing said memory B cells to
(i) at least one first molecular beacon which fluoresces upon hybridization with at least one first target RNA sequence encoding the constant region of the immunoglobulin; and/or
(ii) at least one second molecular beacon which fluoresces upon hybridization with at least one second target RNA sequence encoding a surrogate marker for an antigen activated memory B cell;
(c) quantifying the level of fluorescence in said memory B cell, wherein the presence or increased level of fluorescence is indicative for the status of an antigen activated memory B cell;
(d) selecting an antigen activated memory B cell determined in step (c); and
(e) cloning the cDNA from the selected memory B cell by single cell reverse transcription polymerase chain reaction (scRT-PCR).
